Europäisches Patentamt

European Patent Office

Office européen des brevets

(11) Veröffentlichungsnummer: **0 085 743**

**A1**

## (12) EUROPÄISCHE PATENTANMELDUNG

(21) Anmeldenummer: 82107783.1

(22) Anmeldetag: 25.08.82

(51) Int. Cl.³: **A 61 N 1/04**

(30) Priorität: 04.02.82 DE 3203759

(43) Veröffentlichungstag der Anmeldung:
17.08.83 Patentblatt 83/33

(84) Benannte Vertragsstaaten:
CH DE FR GB IT LI NL SE

(71) Anmelder: W.C. Heraeus GmbH
Heraeusstrasse 12 - 14
D-6450 Hanau / Main(DE)

(72) Erfinder: Aldinger, Fritz, Dr., Dipl.-Ing.
Barbarossastrasse 44
D-6458 Rodenbach 2(DE)

(72) Erfinder: Becker, Wolfgang
Leibziger Strasse 33
D-6480 Wächtersbach 6(DE)

(72) Erfinder: Werdecker, Waltraud, Dipl.-Ing.
Gustav-Hoch-Strasse 11
D-6450 Hanau(DE)

(72) Erfinder: Eckl, Ludwig
Varangeviller Strasse 17a
D-6454 Bruchköbel(DE)

(72) Erfinder: Pieper, Werner
Varangeviller Strasse 2a
D-6454 Bruchköbel(DE)

(74) Vertreter: Heinen, Gerhard
Heraeusstrasse 12-14
D-6450 Hanau/Main(DE)

(54) Stimulationselektrode und Verfahren zu deren Herstellung.

(57) Es wird eine Stimulationselektrode, insbesondere Herzschrittmacherelektrode, beschrieben, deren Elektrodenkopf (1) aus miteinander versinterten Kügelchen oder kugelähnlichen Granulatteilchen (4) besteht, die einen Durchmesser im Bereich von 5 bis 400 μm besitzen. Mit dem Elektrodenkopf (1) ist ein Stift (2) oder ein Röhrchen (5) unverrückbar verbunden. Die Kügelchen (4), der Stift (2) oder das Röhrchen (5) bestehen aus iridiumhaltigem Metall, insbesondere aus Iridium oder Platin-Iridium-Legierungen. Die Herstellung der Elektrode erfolgt in der Weise, daß die Kügelchen in eine den äußeren Abmessungen eines Elektrodenkopfes entsprechende Form eingeführt werden. Die gefüllte Form wird in einen Ofen eingebracht. Die Kügelchen werden in dem Ofen unter Schutzgasatmosphäre oder unter Vakuum an den Berührungsstellen miteinander versintert.

Fig 1a

- 6 -

Hanau, den 29. Jan. 1982
ZPL-Dr.Hn/W

W. C. Heraeus GmbH, Hanau

Patent- und Gebrauchsmusterhilfsanmeldung

"Stimulationselektrode und Verfahren zu deren
Herstellung"

Die Erfindung bezieht sich auf eine Stimulationselektrode,
insbesondere Herzschrittmacherelektrode, und ein Verfahren
zu deren Herstellung.

Bekannt sind aus der US-PS 4 011 861 Stimulationselektroden,
die in den menschlichen Körper implantiert werden, insbesondere
Herzschrittmacherelektroden, die einen Elektrodenkopf aus
Iridium-haltigem Metall aufweisen, der wenigstens eine Schicht
aus durch Sintern miteinander verbundenen Granulatteilchen
aus Irdium-haltigem Metall besitzt.

Aufgabe der Erfindung ist es, eine Stimulationselektrode,
insbesondere eine Herzschrittmacherelektrode, zu schaffen,
die ein optimales Einwachsen des Körpergewebes nach der

- 7 -

Implantation ermöglicht, die im implantierten Zustand im
Betrieb eine möglichst geringe elektrische Verlustleistung
besitzt, deren Polarisation möglichst niedrig ist und deren
Reizschwelle einen möglichst kleinen Spannungswert hat.

Gelöst wird diese Aufgabe für eine Elektrode der eingangs
charakterisierten Art erfindungsgemäß dadurch, daß der gesamte
Elektrodenkopf aus miteinander versinterten Kügelchen oder
kugelähnlichen Granulatteilchen besteht, die einen Durchmesser
im Bereich von 5 bis 400 µm besitzen, mit dem ein Stift
oder ein Röhrchen aus Iridium-haltigem Metall unverrückbar
verbunden ist.

Weitere vorteilhafte Merkmale der erfindungsgemäßen Elektrode
ergeben sich aus den Unteransprüchen 2 bis 14.

Für die Herstellung erfindungsgemäßer Elektroden hat sich
ein Verfahren bewährt, wie es in Anspruch 15 charakterisiert
ist. Vorteilhafte Weiterbildungen des Verfahrens ergeben
sich aus den Unteransprüchen 16 bis 20.

Bei den erfindungsgemäßen Elektroden besteht der Elektrodenkopf
aus einem Gerüst aus miteinander versinterten Kügelchen
oder kugelähnlichen Granulatteilchen bestimmter Abmessungen,
wobei insbesondere Kügelchen oder kugelähnliche Granulatteilchen mit einem Durchmesser von 40 bis 60 µm verwendet werden.
Das Porennetzwerk des Elektrodenkopfes besitzt keine Sackgassen, sondern sämtliche Poren sind miteinander verbunden,
wodurch ein gleichmäßiges Einwachsen von Körpergewebe und
eine vollständige Ausfüllung mit Körperelektrolyt im implantierten Zustand sichergestellt ist. Durch die Verwendung von
Kügelchen oder kugelähnlichen Granulatteilchen ist ausgeschlossen, daß eine Gewebeverletzung auftritt, was bei der
Verwendung von scharfkantigen Granulatteilchen gemäß dem
Stand der Technik nicht vollständig vermeidbar ist.

Weiterhin ist als Vorteil der erfindungsgemäß ausgebildeten Elektrode anzuführen, daß durch die Verwendung von Kügelchen oder kugelähnlichen Granulatteilchen die Bildung von Spannungsspitzen vermieden wird, so daß keine Mikroreizung und damit verbundene Gefahren der Bildung von Entzündungen entstehen können. Außerdem ist auf Grund der Porosität eine niedrige Polarisation zu erwarten, die wegen der kugelförmigen Gestalt der Granulatteilchen sehr konstante Werte aufweist. Durch die Verwendung von Kügelchen oder kugelförmigen Granulatteilchen, deren Durchmesser in einem engen Durchmesserbereich liegt, läßt sich die spezifische Oberfläche des Elektrodenkopfes gut berechnen und auch die Anzahl der Stimulationspunkte pro Flächeneinheit gut vorherbestimmen, was sehr nützlich für Voraussagen über die elektrischen Eigenschaften der Elektrode im implantierten Zustand ist.

Der mit dem Elektrodenkopf unverrückbar verbundene Stift bzw. das Röhrchen dient vorzugsweise dazu, die mechanische Stabilisierung der meist in Form einer Drahtspirale ausgebildeten Stromzuführung sicherzustellen, so daß kein Abscheren oder Abknicken der Stromzufuhrleitung an der Anschlußseite des Elektrodenkopfes befürchtet werden muß.

Zur Vermeidung von Lokalelementbildung bestehen der Stift bzw. das Röhrchen vorteilhafterweise aus dem gleichen Werkstoff wie die Kügelchen oder kugelähnlichen Granulatteilchen des Elektrodenkopfes.

Als Werkstoff für die Kügelchen oder kugelähnlichen Granulatteilchen haben sich insbesondere Legierungen Pt97Ir3, Pt90Ir10, Pt80Ir20 und reines Iridium bewährt. Trotz der hohen Dichte dieser Werkstoffe lassen sich erfindungsgemäß ausgebildete Elektroden mit einem Gewicht von weniger als 0,1 g und einem Volumen von etwa 5 mm³ herstellen. Es handelt sich also um außerordentlich kleine Elektroden.

Zur Beeinflussung der Geometrie des elektrischen Feldes
des Elektrodenkopfes hat es sich als vorteilhaft erwiesen,
elektrisch nichtleitende Bereiche, insbesondere an der freien
Stirnfläche des Elektrodenkopfes, vorzusehen. Diese können
aus einem Kanal bestehen oder aus Einlagen, wie sie in den
Unteransprüchen charakterisiert sind.

Das erfindungsgemäße Herstellverfahren stellt sicher, daß
beim Sintervorgang keine wesentliche Schrumpfung auftritt,
jedenfalls keine solche, die mehr als 5 % beträgt. Eine
Nachbearbeitung des nach dem Verfahren hergestellten Elektrodenkopfes ist nicht erforderlich, so daß erfindungsgemäße Elektroden auch kostengünstig herstellbar sind.

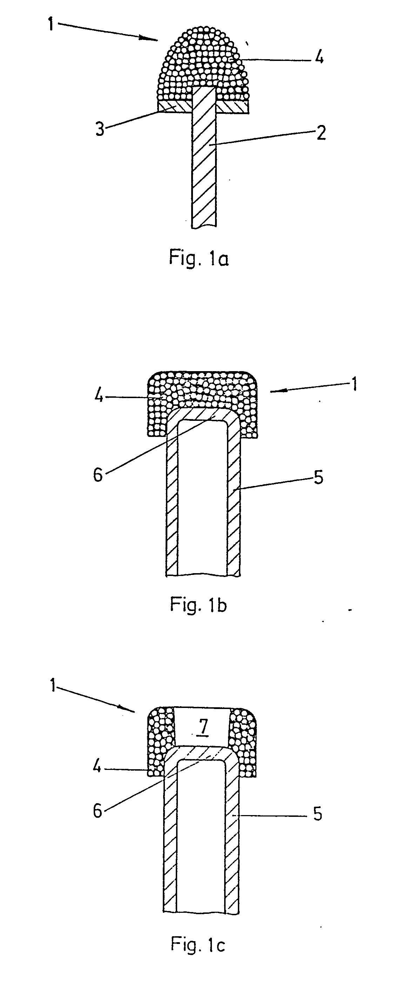

In den Figuren 1a bis 1g sind Ausführungsbeispiele erfindungsgemäßer Elektroden im Vertikalschnitt dargestellt.

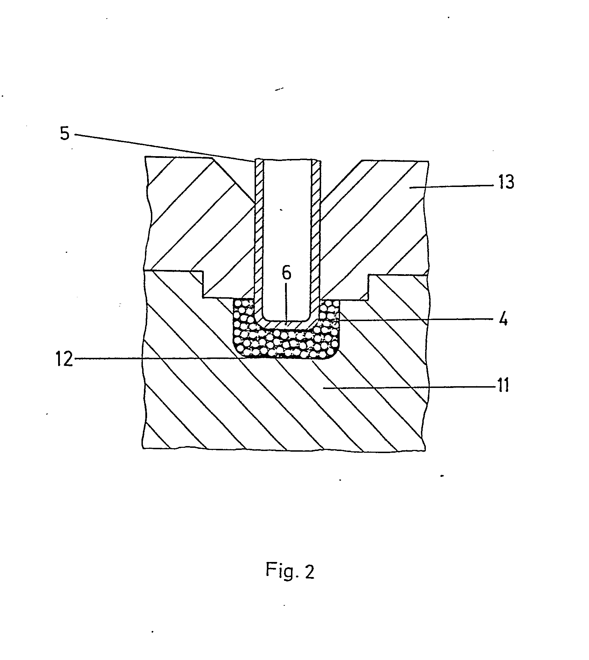

Anhand der Figur 2, die einen Vertikalschnitt durch eine
Sinterform darstellt, wird das erfindungsgemäße Verfahren
erläutert.

Figur 1a zeigt eine Elektrode, die einen Elektrodenkopf 1
aufweist, der aus miteinander versinterten Kügelchen oder
kugelähnlichen Granulatteilchen 4 aus einer Pt-Ir-Legierung
mit 90 Gewichts-% Pt und 10 Gewichts-% Ir besteht. Mit dem
Elektrodenkopf ist unverrückbar ein Stift 2 verbunden sowie
die nicht poröse Platte 3. Der Stift 2 und die Platte 3
bestehen in dem Ausführungsbeispiel aus dem gleichen Werkstoff
wie die Kügelchen oder kugelähnlichen Granulatteilchen 4.

Die in Figur 1b dargestellte Elektrode unterscheidet sich
von der gemäß Figur 1a dadurch, daß der Elektrodenkopf eine
kappenförmige Geometrie aufweist und daß anstelle des Stifts 2 ein Röhrchen 5 unverrückbar mit dem Elektrodenkopf 1 verbunden ist. Das Röhrchen 5 weist ein nicht poröses Verschlußstück bzw. Boden 6 auf.

Bei der Elektrode gemäß Figur 1c ist von der freien Stirnfläche des Elektrodenkopfes 1 her ein Kanal 7 vorgesehen, der zur Beeinflussung des elektrischen Feldes dient.

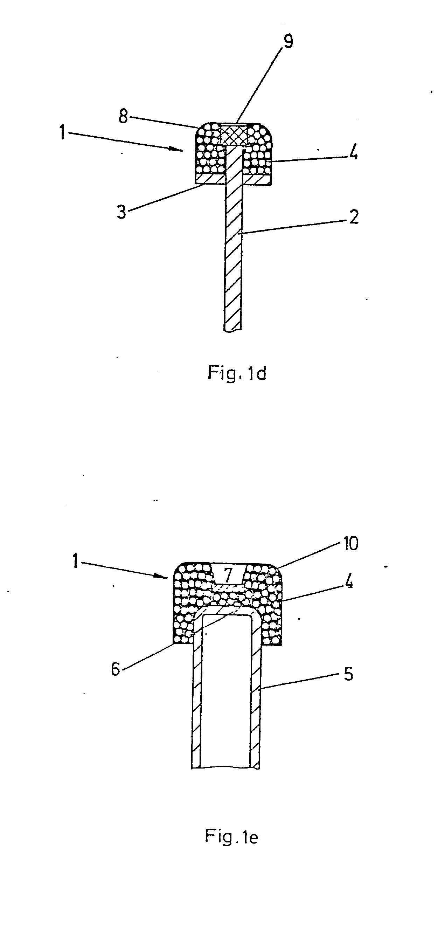

Bei der Elektrode nach Figur 1d ist in eine Ausnehmung in der freien Stirnfläche zur Beeinflussung des elektrischen Feldes des Elektrodenkopfes ein Tantalkörper 8 unverrückbar angeordnet, dessen freie Oberfläche eine Tantaloxidschicht 9 aufweist, die elektrisch nichtleitend ist. Solche Tantaleinlagen können entweder nach dem Versintern der Kügelchen 4 eingebracht werden oder vor dem Versintern der Kügelchen 4 in die Form eingesetzt werden, in die dann die Kügelchen eingefüllt und ggf. eingerüttelt werden.

Die Figur 1e zeigt eine weitere Ausbildung einer erfindungsgemäßen Elektrode. Hierbei ist am Boden der Ausnehmung 7 eine Aluminiumoxidplatte 10 angeordnet.

Anstelle der Aluminiumoxidplatte 10 (Figur 1e) oder des Tantalkörpers (Figur 1d) kann auch ein körperverträglicher Kunststoff, insbesondere aus Polyurethan oder Silikonkautschuk, in die Ausnehmung eingesetzt sein.

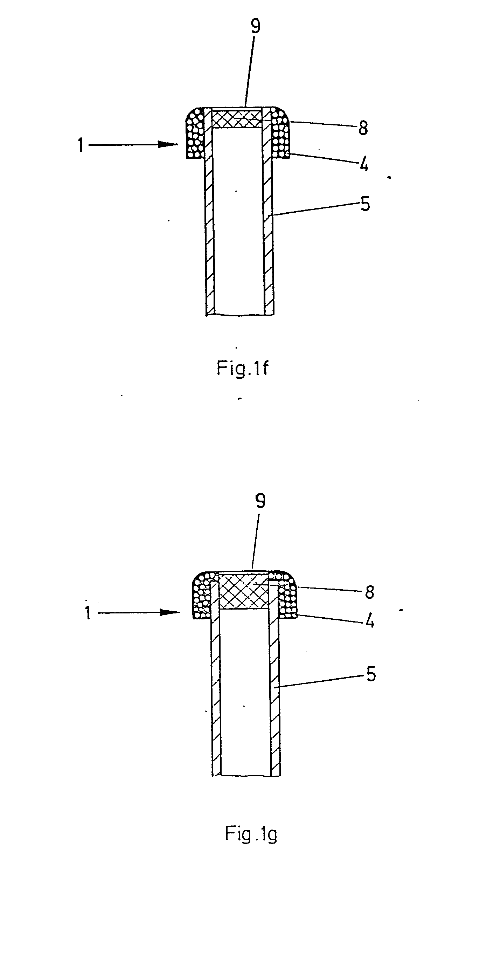

In den Figuren 1f und 1g sind weitere Ausführungsbeispiele erfindungsgemäßer Elektroden dargestellt. Bei der Elektrode nach Figur 1f erstreckt sich die Fassung zur Aufnahme des Röhrchens 5 durch den Elektrodenkopf hindurch bis zu dessen Stirnfläche. In das Vorderende des in die Fassung eingesetzten Röhrchens 5 ist ein Tantalstopfen 8 eingebracht, dessen freies, der Stirnfläche des Elektrodenkopfes zugekehrtes Ende eine Schicht 9 aus Tantaloxid aufweist. Der Tantalstopfen 8

- 11 -

kann entweder vor dem Versintern der Kügelchen 4 oder danach
in das Röhrchen 5 eingebracht werden.

Die in Figur 1g dargestellte Elektrode unterscheidet sich
von der in Figur 1f dargestellten im wesentlichen dadurch,
daß die Fassung sich mit einem etwa dem Außendurchmesser
des Röhrchens 5 entsprechenden Durchmesser bis in die Nähe
der Stirnfläche des Elektrodenkopfes erstreckt und sich
dann mit einem kleineren Durchmesser bis zu dieser Stirnfläche
fortsetzt. Das Röhrchen 5 ragt mit seinem vorderen Ende
bis in die Nähe der Stirnfläche des Elektrodenkopfes, von
der her ein Tantalstopfen 8 teilweise in das Röhrchen 5
hineinragt. Das freie, der Stirnfläche des Elektrodenkopfes
zugekehrte Ende des Tantalstopfens 8 weist wiederum eine
Schicht 9 aus Tantaloxid auf.

Die Herstellung erfindungsgemäßer Elektroden erfolgt in
der Weise, daß wie in Figur 2 dargestellt, eine Hohlform
verwendet wird, vorzugsweise eine Graphitform 11, deren
innerer Bodenbereich 12 der äußeren Form des herzustellenden
Elektrodenkopfes entspricht. In die Hohlform werden
Kügelchen 4, beispielsweise aus einer Legierung aus
80 Gewichts-% Pt und 20 Gewichts-% Ir eingefüllt. In die
Schüttung aus den Kügelchen 4 wird danach ein Röhrchen 5
eingesetzt. Dieses Röhrchen 5 besteht vorteilhafterweise
aus dem gleichen Werkstoff wie die Kügelchen 4. Es wird
in der Form 11 mittels des Fixierteils 13, das ebenfalls
aus Graphit besteht, gehalten. Es empfiehlt sich, danach
diese Anordnung einem Rüttelvorgang zu unterwerfen, um eine
möglichst dichte Packung der Kügelchen 4 zu erzielen und
auch das Röhrchen 5 genügend tief bis zur gewünschten Stelle
eintauchen zu lassen.

Die derart vorbereitete Anordnung wird danach in einen handelsüblichen Ofen eingebracht und unter Schutzgasatmosphäre
oder Vakuum auf Sintertemperatur erhitzt. Nach dem Sintervorgang
werden nach Abkühlung zunächst die Form aus dem Ofen und
aus der Form die bereits fertige Elektrode, wie sie in
Figur 1b dargestellt ist, entnommen.

Es ist auch möglich, nur einen Elektrodenkopf, wie vorstehend
beschrieben, herzustellen ohne Einsatz eines Röhrchens.
Das Fixierteil 13 kann dann ebenfalls entfallen. Es wird
eine Graphithohlform verwendet, deren Aushöhlung direkt
dem herzustellenden Elektrodenkopf entspricht. Nach Entnahme
des Elektrodenkopfes aus der Hohlform nach dem Sintervorgang
wird in diese eine Fassung gebohrt, in die dann ein Stift
oder ein Röhrchen eingesetzt und mit dem Kopf diffusionsverschweißt wird.

Hanau, den 29. Jan. 1982
ZPL-Dr.Hn/W

W. C. Heraeus GmbH, Hanau

Patent- und Gebrauchsmusterhilfsanmeldung

"Stimulationselektrode und Verfahren zu deren Herstellung"

Patentansprüche

1. Stimulationselektrode, insbesondere Herzschrittmacherelektrode, mit einem Elektrodenkopf aus Iridium-haltigem Metall, der wenigstens eine Schicht aus durch Sintern miteinander verbundenen Granulatteilchen aus Iridium-haltigem Metall aufweist, dadurch gekennzeichnet, daß der gesamte Elektrodenkopf aus miteinander versinterten Kügelchen oder kugelähnlichen Granulatteilchen besteht, die einen Durchmesser im Bereich von 5 bis 400 μm besitzen, mit dem ein Stift oder ein Röhrchen aus Iridium-haltigem Metall unverrückbar verbunden ist.

2. Elektrode nach Anspruch 1, dadurch gekennzeichnet, daß die Kügelchen oder kugelähnlichen Granulatteilchen einen Durchmesser von 40 bis 60 μm besitzen.

- 2 -

- 2 -

3. Elektrode nach den Ansprüchen 1 und/oder 2, dadurch gekennzeichnet, daß die Kügelchen, das kugelähnliche Granulat und der Stift oder das Röhrchen aus Iridium oder aus einer Legierung aus Platin und Iridium bestehen, deren Iridiumgehalt entweder 3 bis 30 % oder 97 bis 99,95 % beträgt.

4. Elektrode nach einem der Ansprüche 1 bis 3, dadurch gekennzeichnet, daß der Elektrodenkopf eine Fassung aufweist, in die der Stift oder das Röhrchen eingesetzt ist.

5. Elektrode nach einem oder mehreren der Ansprüche 1 bis 4, dadurch gekennzeichnet, daß die größte Abmessung des Elektrodenkopfes quer zur Achse des Stiftes oder des Röhrchens weniger als 3 mm beträgt.

6. Elektrode nach einem oder mehreren der vorhergehenden Ansprüche, dadurch gekennzeichnet, daß der Stift im Bereich seines dem Elektrodenkopf zugekehrten Endes eine nicht poröse Platte bzw. das Röhrchen an seinem dem Elektrodenkopf zugekehrten Ende ein nicht poröses Verschlußstück (Boden) aufweist.

7. Elektrode nach einem oder mehreren der vorhergehenden Ansprüche, dadurch gekennzeichnet, daß im Elektrodenkopf elektrisch nichtleitende Bereiche vorgesehen sind.

8. Elektrode nach Anspruch 7, dadurch gekennzeichnet, daß die elektrisch nichtleitenden Bereiche an der freien Stirnfläche des Elektrodenkopfes angeordnet sind.

9. Elektrode nach den Ansprüchen 7 und/oder 8, dadurch gekennzeichnet, daß der elektrisch nichtleitende Bereich als Kanal ausgebildet ist.

- 3 -

10. Elektrode nach den Ansprüchen 7 und/oder 8, dadurch gekennzeichnet, daß der elektrisch nichtleitende Bereich aus
einer Einlage besteht, die aus körperverträglicher Keramik,
wie Aluminiumoxid, körperverträglichem Kunststoff oder
aus körperverträglichem Metall mit elektrisch nichtleitender
Oberflächenschicht besteht.

11. Elektrode nach Anspruch 10, dadurch gekennzeichnet, daß
die Einlage aus Tantal besteht, dessen freie Oberfläche
mit Tantaloxid bedeckt ist.

12. Elektrode nach Anspruch 10, dadurch gekennzeichnet, daß
die Einlage aus Polyurethan oder Silikonkautschuk besteht.

13. Elektrode nach Anspruch 4, dadurch gekennzeichnet, daß
die Fassung sich bis zur Stirnfläche durch den Elektrodenkopf hindurch erstreckt und das Vorderende des Röhrchens
bis zu dieser Stirnfläche ragt und wobei das Vorderende
des Röhrchens mit einem Tantalstopfen verschlossen ist,
dessen freies, der Stirnfläche des Elektrodenkopfes zugekehrtes Ende eine Schicht aus Tantaloxid aufweist.

14. Elektrode nach Anspruch 4, dadurch gekennzeichnet, daß
die Fassung sich mit einem etwa dem Außendurchmesser
des Röhrchens entsprechenden Durchmesser bis in die Nähe
der Stirnfläche des Elektrodenkopfes erstreckt und sich
mit einem kleineren Durchmesser bis zu dieser Stirnfläche
fortsetzt und daß von der Stirnfläche her ein Tantalstopfen
teilweise in das in die Fassung eingesetzte Röhrchen
hineinragt, dessen freies, der Stirnfläche des Elektrodenkopfes zugekehrtes Ende eine Schicht aus Tantaloxid
aufweist.

15. Verfahren zur Herstellung einer Stimulationselektrode, insbesondere einer Herzschrittmacherelektrode, nach einem oder mehreren der vorhergehenden Ansprüche 1 bis 14, dadurch gekennzeichnet, daß Iridium-haltige Kügelchen oder kugelähnliche Granulatteilchen mit einem Durchmesser von 5 bis 400 µm in eine den äußeren Abmessungen eines Elektrodenkopfes entsprechende Form eingefüllt und erforderlichenfalls gerüttelt werden, die gefüllte Form in einen Ofen eingebracht und unter Schutzgasatmosphäre oder unter Vakuum die Kügelchen oder kugelähnlichen Granulatteilchen an ihren Berührungsstellen miteinander versintert werden.

16. Verfahren nach Anspruch 15, dadurch gekennzeichnet, daß nach der Entnahme des Elektrodenkopfes aus der Form eine Fassung in den Elektrodenkopf gebohrt wird, in die ein Iridium-haltiger Stift oder ein Röhrchen eingesetzt und mit dem Kopf unverrückbar verbunden wird.

17. Verfahren nach Anspruch 15, dadurch gekennzeichnet, daß nach dem Einfüllen der Kügelchen oder kugelähnlichen Granulatteilchen in die Form ein Iridium-haltiger Stift oder Röhrchen in die Schüttung aus den Kügelchen oder kugelähnlichen Granulatteilchen eingesetzt und nach dem Fixieren eingerüttelt wird und daran anschließend die Kügelchen oder kugelähnlichen Teilchen miteinander und mit dem Stift oder dem Röhrchen versintert werden.

18. Verfahren nach einem der Ansprüche 15 bis 17, dadurch gekennzeichnet, daß eine Graphitform verwendet wird.

19. Verfahren nach einem oder mehreren der Ansprüche 15 bis 18, dadurch gekennzeichnet, daß vor dem Einfüllen der Kügelchen oder kugelähnlichen Granulatteilchen in die Form zur Bildung eines elektrisch nichtleitenden Bereichs im Elektrodenkopf ein Keramikkörper oder ein

- 5 -

Körper aus Metall, der eine elektrisch nichtleitende
Oberflächenschicht aufweist, eingebracht wird.

20. Verfahren nach einem oder mehreren der Ansprüche 15
bis 18, dadurch gekennzeichnet, daß nach der Entnahme
des Elektrodenkopfes aus der Form in eine Ausnehmung
an der freien Stirnfläche des Elektrodenkopfes ein Körper
aus Aluminiumoxid, Polyurethan oder Silikonkautschuk
oder aus Tantal mit einer Tantaloxidoberflächenschicht
unverrückbar eingesetzt wird.

Fig. 1a

Fig. 1b

Fig. 1c

0085743

Fig. 1d

Fig.1e

3/4

Fig.1f

Fig.1g

Fig. 2

## EINSCHLÄGIGE DOKUMENTE

EP 82107783.1

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A,D | <u>US - A - 4 011 861</u> (ENGER) <br> & DE-A1-2 702 240 <br><br> -- | | A 61 N 1/04 |
| A | <u>DE - A1 - 3 017 284</u> (BISPING) <br> * Ansprüche 1,3,6,19; Seite 11, Zeilen 14-27 * <br><br> -- | 4,7-9 | |
| A | <u>DE - A1 - 2 827 595</u> (CARDIAC) <br> * Ansprüche 1,5,6; Seite 10, Zeilen 6-7, 21-25; Seite 11, Zeilen 1-12; Fig. 3-5 * <br><br> -- | 1-3,15 | |
| A | <u>DE - A1 - 2 613 086</u> (SIEMENS) <br> * Seite 5, Zeilen 1-9 * <br><br> -- | 1,15 | |
| A | <u>DE - A - 2 319 054</u> (LAGERGREN) <br> * Ansprüche 1,3,11; Fig. 1-3 * <br><br> -- | 1 | |
| A | <u>US - A - 4 281 669</u> (MACGREGOR) <br> * Spalte 1, Zeilen 48-57; Fig. 1 * <br><br> -- | 1,2 | |
| A | <u>US - A - 4 280 514</u> (MACGREGOR) <br> * Zusammenfassung; Spalte 2, Zeilen 13-38; Fig. 3,4 * <br><br> -- | 1,2 | |

**RECHERCHIERTE SACHGEBIETE (Int. Cl. ³)**

A 61 N
A 61 B
G 01 N
H 01 M

Der vorliegende Recherchenbericht wurde für alle Patentansprüche erstellt.

| Recherchenort | Abschlußdatum der Recherche | Prüfer |
|---|---|---|
| WIEN | 28-12-1982 | NEGWER |

| Kategorie | Kennzeichnung des Dokuments mit Angabe, soweit erforderlich, der Maßgeblichen Teile | betrifft Anspruch | KLASSIFIKATION DER ANMELDUNG (Int. Cl.³) |
|---|---|---|---|
| | **EINSCHLÄGIGE DOKUMENTE** | | |
| A | US - A - 4 125 116 (FISCHELL) <br> * Zusammenfassung; Spalte 1, Zeilen 42-44, 48-50; Spalte 2, Zeilen 9-41; Fig. 1 * <br> -- | 1,7,8, 10 | |
| A | US - A - 3 981 309 (CANNON) <br> * Zusammenfassung; Fig. 3, Spalte 3, Zeile 24 - Spalte 4, Zeile 10 * <br> ---- | 1,2,15 | |
| | | | **RECHERCHIERTE SACHGEBIETE (Int. Cl.³)** |

EPA Form 1503.2  06.78